# FASCICULE DE BREVET EUROPEEN

(11) **EP 2 352 839 B1**
(45) Date de publication et mention de la délivrance du brevet: **25.02.2015**
(21) Numéro de dépôt: 09756026.2
(22) Date de dépôt: 14.10.2009
(51) Int. Cl.: C12Q 1/04

(54) **MILIEU REACTIONNEL POUR LA DETECTION ET/OU L'IDENTIFICATION DE BACTERIES DU GENRE LEGIONELLA**
REAKTIONSMEDIUM ZUM NACHWEISEN UND/ODER IDENTIFIZIEREN VON BAKTERIEN DER GATTUNG LEGIONELLA
REACTION MEDIUM FOR DETECTING AND/OR IDENTIFYING BACTERIA OF THE LEGIONELLA GENUS

(30) Priorité: 17.10.2008 FR 0857078
(43) Date de publication de la demande: 10.08.2011
(73) Titulaire: Biomérieux, 69280 Marcy L'etoile (FR)
(72) Inventeur: CELLIER, Marie, F-38390 Montalieu-Vercieu (FR); ORENGA, Sylvain, F-01160 Neuville sur Ain (FR); ROBICHON, Denis, F-01150 Blyes (FR); SAUVONNET, Véronique, F-38510 Courtenay (FR)
(86) Numéro de dépôt international: PCT/FR2009/051961
(87) Numéro de publication internationale: WO 2010/043818

(56) Documents cités:
- US-A1- 2004 029 212
- HU XIU-RONG ET AL: "[Study on the mechanism of the interaction between montmorillonite and bacterium]" YAO XUE XUE BAO = ACTA PHARMACEUTICA SINICA SEP 2002 (ABSTRACT), vol. 37, no. 9, septembre 2002 (2002-09), pages 718-720, XP002526566 ISSN: 0513-4870
- FEELEY J C ET AL: "Charcoal-yeast extract agar: primary isolation medium for Legionella pneumophila." JOURNAL OF CLINICAL MICROBIOLOGY OCT 1979, vol. 10, no. 4, octobre 1979 (1979-10), pages 437-441, XP002568475 ISSN: 0095-1137
- EDELSTEIN P H: "Improved semiselective medium for isolation of Legionella pneumophila from contaminated clinical and environmental specimens" JOURNAL OF CLINICAL MICROBIOLOGY 1981 US, vol. 14, no. 3, 1981, pages 298-303, XP002526554 ISSN: 0095-1137
- LEE TZIELAN C ET AL: "Growth of 28 Legionella species on selective culture media: A comparative study" JOURNAL OF CLINICAL MICROBIOLOGY, vol. 31, no. 10, 1993, pages 2764-2768, XP002526555 ISSN: 0095-1137
- EDELSTEIN PAUL H ET AL: "Comparison of three buffers used in the formulation of buffered charcoal yeast extract medium" JOURNAL OF CLINICAL MICROBIOLOGY, vol. 31, no. 12, 1993, pages 3329-3330, XP002526556 ISSN: 0095-1137
- MORRILL W E ET AL: "INCREASED RECOVERY OF LEGIONELLA-MICDADEI AND LEGIONELLA-BOZEMANII ON BUFFERED CHARCOAL YEAST EXTRACT AGAR SUPPLEMENTED WITH ALBUMIN" JOURNAL OF CLINICAL MICROBIOLOGY, vol. 28, no. 3, 1990, pages 616-618, XP002526557 ISSN: 0095-1137

## Description

La présente invention concerne un milieu réactionnel pour les légionelles, ainsi que l'utilisation d'un tel milieu, et un procédé mettant en oeuvre ce milieu.

Les légionelles (*Legionella sp*) sont des bactéries de l'environnement hydrique (écosystèmes naturels et réseaux de distribution d'eaux) susceptibles de provoquer des infections, parfois mortelles chez l'homme. Plusieurs espèces du genre *Legionella* ont été mises en évidence chez l'homme, mais la plus importante est *Legionella pneumophila* (*L. pneumophila*), responsable d'environ 90% des cas de légionelloses. D'autres espèces telles que *L*.*jordanis* sont généralement isolées chez des sujets immunodéprimés. La légionellose (ou maladie du légionnaire) est une maladie respiratoire caractérisée par une pneumopathie aiguë sévère, dont l'infection est acquise par l'inhalation d'aérosols contaminés par les *Legionella* comme les tours de refroidissement, les systèmes de climatisation, les établissements thermaux, les douches, etc ... Après une période d'incubation de 2 à 10 jours, les patients présentent un syndrome pseudogrippal. Il s'ensuit une fièvre élevée, une pleurésie, une toux importante, associées à des troubles gastro-intestinaux (diarrhée, vomissements) voire parfois neurologiques (délire, somnolence, confusion). En 2005, plus de 1 500 cas ont été signalés en France et environ 5 700 en Europe ; aux Etats-Unis, le CDC (Center for Disease Control and Prevention) estime qu'il y aurait 8 000 à 18 000 cas de légionellose par an.

Le diagnostic de la légionellose est très complexe car la maladie est atypique : classique d'une pneumopathie, elle n'a rien de spécifique. La mortalité est de 20% des cas en moyenne, plutôt faible dans les cas communautaires mais en revanche, importante dans les cas nosocomiaux. Un diagnostic précoce est essentiel pour proposer au patient un traitement adapté.

Le diagnostic peut être réalisé selon les méthodes suivantes :
- l'identification par une méthode d'immunofluorescence directe. Réalisée sur prélèvement avec des anticorps des principaux sérogroupes de *L. pneumophila,* cette méthode permet la mise en évidence des antigènes solubles. Les méthodes utilisées principalement sont l'ELISA (Enzyme Linked ImmunoSorbant Assay), la RIA (RadioImmunology Assay) ou l'ICM (ImmunoChromatographie sur Membrane). L'inconvénient majeur de ces techniques est qu'elles ne permettent de détecter que la présence éventuelle de certains sérogroupes de *L. pneumophila.*
- le diagnostic sérologique des anticorps anti-Antigène O du LPS par immunofluorescence indirecte. Cette méthode ne permet d'identifier que *L. pneumophila* sérogroupe 1 et présente le risque de nombreux faux positifs dus aux réactions croisées avec les mycobactéries, les leptospires, *Chlamydia*, *Mycoplasma, Pseudomonas, Flavobacterium* ...
- l'identification bactériologique par la méthode de culture. La croissance des légionelles est difficile. En effet, elles ne cultivent pas sur géloses au sang, le pH doit être strictement contrôlé (6,9+/-0,2) et les exigences de la bactérie remplies. Ainsi, les cultures sont généralement réalisées sur une gélose BCYE (Buffered Charcoal Yeast Extract) supplémentée en tampon ACES, en L-Cystéine, et en Fer ; ces deux derniers éléments étant des facteurs de croissance indispensables ; ou GVPC. Les colonies sont observables dès 48h d'incubation à 37°C. L'identification des légionelles peut être réalisée sur milieu *Legionella* GVPC (bioMérieux), qui est un milieu spécifique et sélectif favorisant l'isolement de la plupart des espèces de *Legionella,* notamment *L. pneumophila.* Ce milieu permet une inhibition de la croissance des bactéries à Gram-positif, de la plupart des bactéries à Gram-négatif, des levures et des moisissures, grâce à un mélange optimisé de trois antibiotiques.
Toutefois, les milieux de culture actuels contiennent du charbon activé pour adsorber les composés toxiques produits par les *Legionella,* ou présents dans le milieu (extrait de levure, agar...), ou par l'autoclavage du milieu de culture qui engendre la formation de radicaux libres, également toxiques pour les légionelles. Ces milieux sont de ce fait d'une couleur noire, qui les rend mal adaptés à l'incorporation de substrats enzymatiques chromogènes ou fluorescents, qui pourraient pourtant faciliter la lecture du milieu.

L'invention se propose de résoudre les problèmes de l'état de la technique en présentant un nouveau milieu réactionnel pour les bactéries du genre *Legionella.*

D'une manière surprenante, les inventeurs ont mis en évidence que l'utilisation de composés siliciés dans un milieu réactionnel permettait une détection rapide et aisée des *Legionella.*

Avant de présenter l'invention, les définitions suivantes sont données pour permettre de mieux comprendre l'invention. Elles ne sont nullement limitatives.

Par milieu réactionnel, on entend un milieu comprenant tous les éléments nécessaires à l'expression d'un métabolisme et/ou à la croissance de microorganismes telles que les légionelles.

Le milieu réactionnel peut être solide, semi-solide ou liquide. Par milieu solide, on entend par exemple un milieu gélifié. L'agar est l'agent gélifiant traditionnel en microbiologie pour la culture des microorganismes, mais il est possible d'utiliser de la gelrite, de la gélatine, de l'agarose ou d'autres gélifiants naturels ou artificiels. Le milieu réactionnel selon l'invention doit permettre la croissance des légionelles.

Le milieu réactionnel peut comprendre un ou plusieurs éléments en combinaison, tels que des acides aminés, des hydrates de carbone, des nucléotides, des minéraux, des vitamines...

Le milieu peut comprendre également un colorant. A titre indicatif, on peut citer comme colorant le bleu d'Evans, du rouge neutre, un opacifiant tel que l'oxyde de Titane, de la Nitroaniline, du vert malachite, du vert brillant, un ou plusieurs indicateurs métaboliques, un ou plusieurs régulateurs métaboliques ...

Le milieu réactionnel peut être un milieu de culture, un milieu de révélation ou un milieu de culture et de révélation. Dans le cas d'un milieu de révélation, la culture des microorganismes est effectuée avant ensemencement et, dans le deuxième cas, le milieu de révélation constitue également le milieu de culture.

L'homme du métier peut également utiliser une bi-boîte, permettant de comparer aisément deux milieux, comprenant différents substrats ou différents mélanges sélectifs, sur lesquels on aura déposé un même échantillon biologique.

Par mélange sélectif, on entend un mélange de composés permettant la culture sélective de microorganismes particuliers. Dans le cadre de la présente invention, le mélange sélectif peut permettre la culture sélective des légionelles. Un tel milieu comprend notamment les composés suivants : Glycine, Sulfate de polymyxine B, Vancomycine ou céfamandole, Cycloheximide ou Anisomycine ou encore natamycine, seuls ou en combinaison.

Le milieu sélectif peut permettre également la culture sélective de certaines espèces de légionelles comme par exemple *L. pneumophila.* Dans ce cas, le mélange sélectif comprend notamment les composés suivants : Glycine, Sulfate de polymyxine B, Vancomycine ou céfamandole, Cycloheximide, Anisomycine, natamycine, Pourpre de Bromocrésol, Bleu de Bromothymol seuls ou en combinaison.

Par composé silicié on entend tout composé contenant l'élément silicium. On peut citer de manière non limitative les zéolithes naturelles ou synthétiques, les argiles, de manière préférentielle l'illite, la montmorillonite et préférentiellement la sépiolite, ou encore les silices apolaires.

Par silice apolaire, on entend une ou plusieurs particules de silice sur lesquelles on a greffé des fonctions chimiques apolaires le plus souvent des chaînes alkylées ayant au moins 2 atomes de carbone. Préférentiellement, elles ont 4 à 24 atomes de Carbone, encore plus préférentiellement 18 atomes de Carbone (octadecyl), 8 (octyl) ou 4 atomes de Carbone (butylique).

Préférentiellement, cette silice apolaire est une octadecyl-silice ou une octyl-silice.

Par substrat enzymatique, on entend une molécule pouvant être métabolisée par une enzyme, en un produit permettant la détection, directe ou indirecte d'un microorganisme. Il peut s'agir de substrat naturel ou synthétique. Le métabolisme du substrat provoquera une variation des propriétés physico-chimiques du milieu réactionnel ou des cellules d'organismes. Cette variation peut être détectée par des méthodes physico-chimiques, notamment des méthodes optiques par l'oeil de l'opérateur ou à l'aide d'instruments, spectrométriques, électriques, magnétiques, ... Préférentiellement, il s'agira d'une variation des propriétés optiques, telles qu'une modification d'absorption, de fluorescence ou de luminescence.

Par substrat enzymatique fluorogène ou chromogène, on entend une molécule dont le métabolisme génère un produit ayant une fluorescence ou une couleur différente du substrat.

Dans le cadre de la présente invention, le substrat enzymatique est préférentiellement choisi parmi les substrats d'oxydo-réductase, d'hydrolase.

Préférentiellement, ce substrat est un substrat de nitroréductase, d'oxydo-réductase, acétoacétyl-CoA réductase, de déshydrogénase, de superoxide dismutase, d'osidase, de peptidase, de nucléase, d'estérase.

Comme substrat chromogène, on peut citer notamment ceux à base d'alizarine, les substrats d'alpha-glucosidase et tout particulièrement l'alizarine-alpha-glucoside Comme substrat fluorogène, on peut citer ceux à base de coumarine, ceux décrits dans le brevet EP 0641351 ("Enzymatic analysis using substrates that yield fluorescent precipitates" HAUGLAND *et al*.*)* ou dans la demande de brevet FR07/55371, les substrats de réductase et notamment ceux de nitroréductase et plus préférentiellement ceux décrits dans la demande de brevet FR07/55373 ou dans le brevet EP 1124986. Comme substrat d'activité enzymatique d'oxydo-réductase, on peut citer plus particulièrement les substrats de réductase et préférentiellement les substrats de nitroréductase.

L'introduction d'un deuxième substrat enzymatique et notamment un deuxième substrat d'alpha-glucosidase dans un milieu contenant déjà de l'alizarine-alpha-glucoside permet d'accroître la différenciation des légionelles des autres genres amenés à pousser sur le milieu.

Par échantillon biologique, on entend un échantillon clinique, issu d'un prélèvement d'aspiration bronchique, trachéale ou pulmonaire, de liquide pleural, d'un lavage broncho-alvéolaire, d'expectorations, du sang ou d'une biopsie pulmonaire et plus rarement de liquide articulaire ou péricardique ; un échantillon alimentaire. Cet échantillon peut être ainsi liquide ou solide et on peut citer d'une manière non limitative, un échantillon clinique de sang, de plasma, d'urines, de fèces, de prélèvements de nez, de gorges, de peaux, de plaies, de liquide céphalo-rachidien, un échantillon alimentaire d'eau (eau potable).

Par échantillon de l'environnement, on entend un prélèvement d'eau, et on peut citer d'une manière non limitative : réseau d'alimentation en eau, système de climatisation, tour aéro-réfrigérante, vaporisateur, brumisateur.

A ce titre, l'invention concerne en premier lieu un milieu réactionnel pour la culture et/ou la détection et/ou l'identification de bactéries du genre *Legionella,* comprenant au moins une silice apolaire. Préférentiellement, ladite silice apolaire est l'octadecyl-silice. Préférentiellement, ladite silice apolaire est en combinaison avec une argile, préférentiellement la sépiolite. Préférentiellement, l'octadecyl-silice est en combinaison avec la sépiolite.

Selon un mode préféré de réalisation de l'invention, ledit milieu réactionnel comprend, en outre, un substrat enzymatique.

Préférentiellement, ledit substrat enzymatique est un substrat enzymatique fluorogène ou chromogène, préférentiellement chromogène.

Préférentiellement, ledit substrat enzymatique fluorogène ou chromogène est un substrat d'oxydo-réductase ou un substrat d'hydrolase.

Selon un mode préféré de réalisation de l'invention, la concentration en silice apolaire est comprise entre 0,01 et 100 g/l, préférentiellement entre 1 et 5 g/l. Dans le cas où le milieu selon l'invention comprend une silice apolaire en combinaison avec une argile, la concentration en argile est également comprise entre 0,01 et 100 g/l, préférentiellement entre 1 et 5 g/l.

Selon un mode préféré de réalisation de l'invention, le milieu réactionnel comprend en outre un mélange sélectif permettant la culture sélective de bactéries du genre *Legionella.* Préférentiellement, ce mélange sélectif comprend de la Glycine, Sulfate de polymyxine B, Vancomycine, Cycloheximide, Anisomycine, Pourpre de Bromocrésol, Bleu de Bromothymol seuls ou en combinaison.

L'utilisation d'un tel milieu permet la détection sélective de bactéries du genre *Legionella.*

Le milieu selon l'invention peut également comprendre un mélange sélectif permettant la culture sélective d'espèces particulières de *Legionella* telle que *Legionella pneumophila.* Ce mélange sélectif comprend alors préférentiellement de la Glycine, Sulfate de polymyxine B, Vancomycine, une Quinolone ou une Fluoroquinolone, Cycloheximide, Anisomycine, Pourpre de Bromocrésol, Bleu de Bromothymol seuls ou en combinaison. La différence de sélectivité peut également être obtenue par la variation de la concentration en Glycine allant de 2,5g/l à 7,5g/l (Glycine-containing selective medium for isolation of Legionellaceae from environmental specimens, Robert M. Wadowski and Robert B. Yee).

L'invention concerne également l'utilisation d'un milieu réactionnel comprenant au moins un composé silicié, pour la culture, la détection et/ou l'identification de bactéries du genre *Legionella.* Préférentiellement, ledit composé silicié est une silice apolaire.

Préférentiellement, ladite silice apolaire est l'octadecyl-silice.

Selon un mode préféré de réalisation de l'invention, la concentration en composé silicié, dans le milieu réactionnel utilisé, est comprise entre 0,01 et 100 g/l, préférentiellement entre 1 et 5 g/l. Dans le cas où le milieu réactionnel utilisé comprend une combinaison de composés siliciés, la concentration de chacun des composés est comprise entre 0,01 et 100 g/l, préférentiellement entre 1 et 5 g/l.

L'invention concerne enfin un procédé de détection et/ou d'identification de bactéries du genre *Legionella,* caractérisé en ce qu'il comprend les étapes suivantes :
a) mettre en contact un échantillon susceptible de contenir des bactéries du genre *Legionella* avec un milieu réactionnel tel que défini ci-avant ;
b) incuber, et,
c) détecter la présence de bactéries du genre *Legionella*

L'incubation est préférentiellement réalisée à une température comprise entre 30°C et 50°C, et plus préférentiellement entre 36°C et 42°C. Les légionelles sont préférentiellement détectées par une activité alpha-glucosidase ou oxydo-réductase qui permet d'obtenir des colonies colorées ou fluorescentes. Les colonies des autres genres, si non inhibés, apparaissent soit incolores soit d'une couleur différente soit d'une couleur ou fluorescence identiques à celles de *Legionella .*

Le temps d'incubation permet la croissance des bactéries du genre *Legionella* pour permettre leur détection. Sans être limitatif, une incubation de 48-72h est bien adaptée, mais une incubation plus courte est possible.

Lors de l'utilisation de ce milieu, les légionelles sont préférentiellement détectées par une activité oxydo-réductase qui permet d'obtenir des colonies colorées ou fluorescentes. Les autres bactéries sont soit inhibées, soit incolores, soit d'une couleur ou fluorescence différente, soit d'une couleur ou fluorescence identique à celles des légionelles.

Les exemples ci dessous sont donnés à titre explicatif et n'ont aucun caractère limitatif. Ils permettront de mieux comprendre l'invention.

### Exemple 1

Différentes souches du genre *Legionella* ont été testées sur 5 milieux différents. La lecture des boîtes est ensuite effectuée après 72h et 96h d'incubation..

### 1. MILIEUX ET MICROORGANISMES

Le milieu de composition suivante a été utilisé (composition en g/l) :
- extrait de levure : 10 g/l
- acide alpha-cétoglutarique : 1 g/l
- ACES/KOH : 4/1,65 g/l
- Glycine : 3 g/l
- Cystéine hydrochloride : 0,4 g/l
- Pyrrophosphate ferrique : 0,25 g/l
- Glutathion : 5 g/l
- Agar : 17 g/l

A ce milieu ont été rajoutés 5 g/l de silice apolaire (octadecyl-silice, milieu 1), 5 g/l de silice polaire (Silica gel, milieu 2), 5g/l de silice très polaire (3-aminopropyl-silice, milieu 3), ou 5 g/l de silice apolaire (octyl-silice, milieu 4).

Le milieu BCYE (Feeley et al, J Clin Microbiol. 1979 10:437-41) était un milieu servant de témoin de croissance.

### 2. ESSAIS

Les milieux étaient répartis en boîtes de Petri. L'inoculation des différentes souches de *Legionella* était effectuée par isolement en trois cadrans. Les boîtes étaient incubées 96h à 37°C en présence de CO₂.

Des lectures sont effectuées après 72 et 96h d'incubation.

### 3. RESULTATS

Les résultats sont consignés dans le tableau ci-après :

| | | BCYE | | milieu 1 | | milieu 2 | | milieu 3 | | milieu 4 | |
|---|---|---|---|---|---|---|---|---|---|---|---|
| | | Croissance | | Croissance | | Croissance | | Croissance | | Croissance | |
| | | M | C | M | C | M | C | M | C | M | C |
| *L. pneumophila* | 72h | 3 | 0,5+ | 3 | 1 | 2 | 0 | 2 | 0 | 3 | 1 |
| 07 10 139 | 96h | 3 | 1 | 3 | 1.25 | 2 | 0 | 2 | 0 | 3 | 1 |
| *L. pneumophila* | 72h | 3 | 0.5 | 3 | 0.75 | 3 | 0.25 | 3 | 0.25 | 3 | 0.75 |
| 07 10 146 | 96h | 3 | 1 | 3 | 1 | 3 | 1 | 3 | 1 | 3 | 1 |
| *L. pneumophila* | 72h | 3 | 0.75 | 3 | 1 | 1 | 0.75 | 1 | 0 | 3 | 1 |
| 07 10 138 | 96h | 3 | 1 | 3 | 1.25 | 2 | 1.25 | 1+ | 0 | 3 | 1 |
| *L. pneumophila* | 72h | 3 | 1 | 3 | 1.25 | 3 | 0.5 | 3 | 0.5 | 3 | 1 |
| 07 10 142 | 96h | 3 | 1.25 | 3 | 1.25 | 3 | 0,5+ | 3 | 0,5+ | 3 | 1 |
| *L. pneumophila* | 72h | 3 | 0.75 | 3 | 1 | voile | | 0 | 0 | 3 | 1 |
| 07 10 144 | 96h | 3 | 1.25 | 3 | 1 | voile | | voile | | 3 | 1 |
| *L. pneumophila* | 72h | 3 | 0.75 | 3 | 1.25 | 3 | 0.1 | 3 | 0.1 | 3 | 1.25 |
| 07 10 143 | 96h | 3 | 1 | 3 | 1.25 | 3 | 0.5 | 3 | 0.5 | 3 | 1.25 |
| *L. erythra* | 72h | 3 | 0.5 | 3 | 0.75 | 0 | 0 | 0 | 0 | 3 | 0,5+ |
| 04 12 069 | 96h | 3 | 0.5 | 3 | 1 | 0 | 0 | 0 | 0 | 3 | 0,5+ |
| *L. rubrilucens* | 72h | 3 | 0,5+ | 3 | 1 | voile | | 0 | 0 | 3 | 1 |
| 04 12 075 | 96h | 3 | 0.75 | 3 | 1 | 2 | 0 | voile | | 3 | 1 |
| *L. feelii* | 72h | 3 | 0.25 | 3 | 1 | 0 | 0 | 0 | 0 | 3 | 1 |
| 04 12 070 | 96h | 3 | 0.25 | 3 | 1 | 0 | 0 | 0 | 0 | 3 | 1 |
| *L. longbeachae* | 72h | 3 | 1 | 3 | 1 | 0 | 0 | 0 | 0 | 3 | 1 |
| 04 12 073 | 96h | 3 | 1 | 3 | 1 | 0 | 0 | 0 | 0 | 3 | 1 |

| | | | | | | | | | | | |
|---|---|---|---|---|---|---|---|---|---|---|---|
| M : masse ; C : colonies | | | | | | | | | | | |

La notation « 1 », « 2 » ou « 3 » dans la colonne M (masse) indique la croissance de la souche testée sur 1, 2 ou 3 cadrans ; « 0 » indique une absence de croissance. La taille des colonies (C) est donnée en millimètres.

### 4. INTERPRETATION

Les milieux contenant des silices de nature apolaire permettaient la croissance de toutes les souches testées, croissance supérieure au milieu BCYE.

### Exemple 2

Différentes souches du genre *Legionella* ont été testées sur 8 milieux différents. La lecture des boîtes est ensuite effectuée après 72h et 96h d'incubation.

### 1. MILIEUX ET MICROORGANISMES

Le milieu de composition suivante a été utilisé (composition en g/l) :
- extrait de levure : 10 g/l
- acide alpha-cétoglutarique : 1 g/l
- ACES/KOH : 4/1,65 g/l
- Glycine : 3 g/l
- Cystéine hydrochloride : 0,4 g/l
- Pyrrophosphate ferrique : 0,25 g/l
- Glutathion : 5 g/l
- Agar : 17 g/l

A ce milieu ont été rajoutés 1 g/l de silice apolaire (octadecyl-silice, milieu T), ou respectivement 0.1, 0.5, 1, 2.5, 5, 7.5, 10g/l de sépiolite (milieux 1, 2, 3, 4, 5, 6, 7)

### 2. ESSAIS

Les milieux étaient répartis en boîtes de Petri. L'inoculation des différentes souches de *Legionella* était effectuée par isolement en trois cadrans. Les boîtes étaient incubées 96h à 37°C en présence de CO₂.

Des lectures sont effectuées après 72 et 96h d'incubation.

### 3. RESULTATS

Les résultats sont consignés dans le tableau ci-après :

| | | T | | 1 | | 2 | | 3 | | 4 | | 5 | | 6 | | 7 | |
|---|---|---|---|---|---|---|---|---|---|---|---|---|---|---|---|---|---|
| | | octadecyl silice g/l | | Sépiolite en g/l | | | | | | | | | | | | | |
| | | 1 | | 0.1 | | 0.5 | | 1 | | 2.5 | | 5 | | 7.5 | | 10 | |
| | | M | C | M | C | M | C | M | C | M | C | M | C | M | C | M | C |
| *L pneumophila* | 72h | 3 | 0.75 | 2 | 0 | 2 | 0 | 2 | 0 | 3 | 0.1 | 3 | 0.75 | 3 | 0.75 | 3 | 0.5 |
| 07 10 139 | 96ho | 3 | 0.75 | 2 | 0 | 2 | 0 | 2 | 0 | 3 | 0.1 | 3 | 0.75 | 3 | 0.75 | 3 | 0.5 |
| *L pneumophila* | 72h | 3 | 1 | 2 | 0 | 2 | 0 | 3 | 0.25 | 3 | 0.25 | 3 | 0.75 | 3 | 0.75 | 3 | 1 |
| 07 10 143 | 96h | 3 | 1.5 | 3 | 0.5 | 3 | 0.75 | 3 | 1 | 3 | 1 | 3 | 1.25 | 3 | 1.25 | 3 | 1.25 |
| *L pneumophila* | 72h | 3 | 0.5 | 2 | 0 | 2 | 0 | 3 | 0.1 | 3 | 0.5 | 3 | 0.75 | 3 | 0.5 | 3 | 0.75 |
| 07 10 138 | 96h | 3 | 0.75 | 2 | 0 | 2 | 0 | 3 | 0.25 | 3 | 0.5 | 3 | 1 | 3 | 0.75 | 3 | 1 |
| *L pneumophila* | 72h | 3 | 0.75 | 3 | 0.5 | 3 | 0.5 | 3 | 0.5 | 3 | 0.75 | 3 | 1 | 3 | 0.75 | 3 | 0.5 |
| 07 10 142 | 96h | 3 | 0.75 | 3 | 1 | 3 | 0.75 | 3 | 1 | 3 | 1.25 | 3 | 1.25 | 3 | 1 | 3 | 1 |
| *L erythra* | 72h | 1 | 0 | 0 | 0 | 0 | 0 | 0 | 0 | 0 | 0 | 0 | 0 | 0 | 0 | 0 | 0 |
| 04 12 069 | 96h | 1 | 0 | 0 | 0 | 0 | 0 | 0 | 0 | 0 | 0 | 0 | 0 | 0 | 0 | 0 | 0 |
| *L rubrilucens* | 72h | 3 | 0.25 | 0 | 0 | 0 | 0 | 1 | 0 | 2 | 0 | 2 | 0 | 3 | 0.1 | 3 | 0.1 |
| 04 12 075 | 96h | 3 | 0.5 | 0 | 0 | 0 | 0 | 1 | 0 | 2 | 0 | 2 | 0 | 3 | 0.75 | 3 | 0.5 |
| *Lfeelii* | 72h | 3 | 0.5 | 0 | 0 | 0 | 0 | 0 | 0 | 2 | 0 | 3 | 0.5 | 3 | 0.5 | 3 | 0.75 |
| 04 12 070 | 96h | 3 | 0.75 | 0 | 0 | 0 | 0 | 1 | 0 | 2 | 0 | 3 | 0.75 | 3 | 0.5 | 3 | 0.75 |
| *L longbeachae* | 72h | 2 | 0 | 0 | 0 | 0 | 0 | 0 | 0 | 0 | 0 | 0 | 0 | 0 | 0 | 0 | 0 |
| 04 12 073 | 96h | 3 | 0.25 | 0 | 0 | 0 | 0 | 0 | 0 | 0 | 0 | 0 | 0 | 0 | 0 | 0 | 0 |

| | | | | | | | | | | | | | | | | | |
|---|---|---|---|---|---|---|---|---|---|---|---|---|---|---|---|---|---|
| Croissance : M : masse ; C : colonies | | | | | | | | | | | | | | | | | |

La notation « 1 », « 2 » ou « 3 » dans la colonne M (masse) indique la croissance de la souche testée sur 1, 2 ou 3 cadrans ; « 0 » indique une absence de croissance. La taille des colonies (C) est donnée en millimètres.

### 4. INTERPRETATION

Les milieux contenant de la sépiolite à des concentrations comprises entre 5 et 10 g/l permettaient une croissance de toutes les *Legionella pneumophila,* croissance sensiblement identique au milieu témoin.

### Exemple 3

Différentes souches du genre *Legionella* ont été testées sur 3 milieux différents. La lecture des boîtes est ensuite effectuée après 72h et 96h d'incubation..

### 1. MILIEUX ET MICROORGANISMES

Le milieu de composition suivante a été utilisé (composition en g/l) :
- extrait de levure : 10 g/l
- acide alpha-cétoglutarique : 1 g/l
- ACES/KOH : 4/1,65 g/l
- Glycine : 3 g/l
- Cystéine hydrochloride : 0,4 g/l
- Pyrrophosphate ferrique : 0,25 g/l
- Glutathion : 5 g/l
- Octadecyl-silice : 1 g/l

A ce milieu ont été rajoutés 17g/l d'agar (milieu T), 17g/l de gelrite (milieu T1) et 5g/l de sépiolite et 17g/l d'agar (milieu T2).

### 2. ESSAIS

Les milieux étaient répartis en boîtes de Petri. L'inoculation des différentes souches de *Legionella* était effectuée par isolement en trois cadrans. Les boîtes étaient incubées 96h à 37°C en présence de CO₂.

Des lectures sont effectuées après 72 et 96h d'incubation.

### 3. RESULTATS

Les résultats sont consignés dans le tableau ci-après :

| | | **T** | | **T1** | | **T2** | |
|---|---|---|---|---|---|---|---|
| Sépiolite g/l | | **0** | | **0** | | **5** | |
| gelrite g/l | | **0** | | **17** | | **0** | |
| Agar g/l | | **17** | | **0** | | **17** | |

| | | Croissance | | Croissance | | Croissance | |
|---|---|---|---|---|---|---|---|
| | | M | C | M | C | M | C |
| *Legionella pneumophila* | 72h | 3 | 1 | 3 | 2 | 3 | 1.25 |
| **07 10 139** | 96h | 3 | 1.25 | 3 | 3 | 3 | 1.25 |
| *Legionella pneumophila* | 72h | 3 | 1.25 | 3 | 1.5 | 3 | 1.25 |
| **07 10 138** | 96h | 3 | 1.5 | 3 | 2 | 3 | 1.25 |
| *Legionella pneumophila* | 72h | 3 | 0.75 | 3 | 1.25 | 3 | 1 |
| **07 10 142** | 96h | 3 | 1 | 3 | 2 | 3 | 1 |
| *Legionella pneumophila* | 72h | 3 | 0.25 | 3 | 1 | 3 | 0.75 |

| | | | | | | | |
|---|---|---|---|---|---|---|---|
| **07 10 147** | 96h | 3 | 1 | 3 | 1.5 | 3 | 1 |
| *Legionella pneumophila* | 72h | 3 | 0.25 | 3 | 1.25 | 3 | 0.75 |
| **07 10 149** | 96h | 3 | 1 | 3 | 1.5 | 3 | 1 |
| *Legionella rubrilucens* | 72h | 3 | 0.25 | 3 | 1.5 | 3 | 0.75 |
| **04 12 075** | 96h | 3 | 0.5 | 3 | 1.5 | 3 | 0.75 |
| *Legionella feelii* | 72h | 2 | 0 | 3 | 1.25 | 3 | 0.25 |
| **04 12 070** | 96h | 3 | 0.25 | 3 | 1.5 | 3 | 0.75 |
| *Legionella longbeachae* | 72h | 3 | 0.1 | 3 | 1 | 3 | 0.5 |
| **04 12 073** | 96h | 3 | 0.75 | 3 | 1.25 | 3 | 0.75 |

La notation « 1 », « 2 » ou « 3 » dans la colonne M (masse) indique la croissance de la souche testée sur 1, 2 ou 3 cadrans. La taille des colonies (C) est donnée en millimètres.

### 4. INTERPRETATION

Tous les milieux testés ici permettaient une croissance de toutes les légionelles avec des tailles de colonies isolées importantes.

### Exemple 4

Différentes souches du genre *Legionella* ont été testées sur un milieu selon l'invention comprenant un substrat fluorogène de nitroréductase, le 2-(5'-fluoro-2'-nitrophenyl)-benzothiazole. La lecture des boîtes est ensuite effectuée après 72h, 96h et 120h d'incubation.

### 1. MILIEUX ET MICROORGANISMES

Le milieu de composition suivante a été utilisé (composition en g/l) :
- extrait de levure : 10 g/l
- acide alpha-cétoglutarique : 1 g/l
- ACES/KOH : 4/1,65 g/l
- Glycine : 3 g/l
- Cystéine hydrochloride : 0,4 g/l
- Pyrrophosphate ferrique : 0,25 g/l
- Glutathion : 5 g/l
- Agar : 17 g/l
- Octadecyl-silice : 1 g/l

Une solution-mère de substrat fluorogène 2-(5'-fluoro-2'-nitrophenyl)-benzothiazole était réalisée dans un solvant type DMSO à raison de 50g/l. Un volume correspondant à une concentration finale de 5mg/l de substrat a été ajouté dans ledit milieu en surfusion.

### 2. ESSAIS

Les milieux ont été coulés en boîtes de Petri d'un diamètre de 55mm puis des souches de *Legionella* ont été ensemencées par isolement en trois cadrans à partir de suspensions à 0,5McFarland. Les boîtes ont été incubées à 37°C pendant 5 jours.

### 3. RESULTATS

Les colonies formées ont été examinées visuellement après 72, 96 et 120 heures d'incubation. La fluorescence (lue sous lampe UV à 366 nm) ainsi que l'intensité ont été notées.

Les résultats sont consignés dans le tableau ci-après :

| | | T | | | | | 1=T+5mg/L en substrat fluorescent de nitroreductase | | | | |
|---|---|---|---|---|---|---|---|---|---|---|---|
| | | Croissance | | Fluorescence | | | Croissance | | Fluorescence | | |
| | | M | C | M | C | Coul, | M | C | M | C | Coul, |
| *Legionella pneumophila* | 72h | 3 | 1.25 | 0 | 0 | 0 | 3 | 0.75 | 0.5 | 0 | Bleue |
| | 96h | 3 | 2- | 0 | 0 | 0 | 3 | 1.5 | 2 | 2 | Bleue |
| 07 10 139 | 120h | 3 | 2+ | 0 | 0 | 0 | 3 | 1.5 | 3 | 3 | Bleue |
| *Legionella pneumophila* | 72h | 3 | 1.25 | 0 | 0 | 0 | 3 | 0.75 | 1 | 0.5 | Bleue |
| | 96h | 3 | 1.25 | 0 | 0 | 0 | 3 | 1 | 2 | 2 | Bleue |
| 07 10 146 | 120h | 3 | 1.5 | 0 | 0 | 0 | 3 | 1.25 | 1 | 3 | Bleue |
| *Legionella pneumophila* | 72h | 3 | 1.5 | 0 | 0 | 0 | 3 | 0.75 | 1 | 0 | Bleue |
| | 96h | 3 | 2 | 0 | 0 | 0 | 3 | 1.5 | 2 | 2 | Bleue |
| 07 10 138 | 120h | 3 | 2 | 0 | 0 | 0 | 3 | 1.5 | 1 | 2 | Bleue |
| *Legionella pneumophila* | 72h | 3 | 1 | 0 | 0 | 0 | 3 | 0.75 | 1 | 0.1 | Bleue |
| | 96h | 3 | 1+ | 0 | 0 | 0 | 3 | 1 | 2 | 2 | Bleue |
| 07 10 142 | 120h | 3 | 1+ | 0 | 0 | 0 | 3 | 1 | 2 | 3 | Bleue |
| *Legionella pneumophila* | 72h | 3 | 0.75 | 0 | 0 | 0 | 3 | 1 | 1 | 0 | Bleue |
| | 96h | 3 | 2- | 0 | 0 | 0 | 3 | 1.25 | 3 | 2 | Bleue |
| 07 10 144 | 120h | 3 | 2 | 0 | 0 | 0 | 3 | 2 | 2 | 3 | Bleue |
| *Legionella pneumophila* | 72h | 3 | 1.5 | 0 | 0 | 0 | 3 | 1 | 0.5 | 0 | Bleue |
| | 96h | 3 | 2 | 0 | 0 | 0 | 3 | 1 | 2 | 2 | Bleue |
| 07 10 143 | 120h | 3 | 2 | 0 | 0 | 0 | 3 | 1.25 | 1 | 3 | Bleue |
| *Legionella erythra* | 72h | 3 | 1 | 0 | 0 | 0 | 1 | 0 | 0.1 | 0 | Bleue |
| | 96h | 3 | 1 | 0 | 0 | 0 | 1 | 1 | 0 | 0.5 | Bleue |
| 04 12 069 | 120h | 3 | 1 | 0 | 0 | 0 | 2 | 1 | 0.1 | 0.5 | Bleue |
| *Legionella rubilucens* | 72h | 3 | 1 | 0 | 0 | 0 | 3 | 0,5+ | 1 | 0 | Bleue |
| | 96h | 3 | 1 | 0 | 0 | 0 | 3 | 0,5+ | 2 | 1 | Bleue |
| 04 12 075 | 120h | 3 | 1 | 0 | 0 | 0 | 3 | 0.75 | 2 | 2 | Bleue |
| *Legionella feelii* | 72h | 3 | 1 | 0 | 0 | 0 | 3 | 0.75 | 1 | 0.1 | Bleue |
| | 96h | 3 | 1 | 0 | 0 | 0 | 3 | 0.75 | 2 | 2 | Bleue |
| 04 12 070 | 120h | 3 | 1 | 0 | 0 | 0 | 3 | 0.75 | 1 | 3 | Bleue |
| *Legionella longbeachae* | 72h | 3 | 1 | 0 | 0 | 0 | 3 | 0.5 | 0.5 | 0 | Bleue |
| | 96h | 3 | 1 | 0 | 0 | 0 | 3 | 0.5 | 0 | 1 | Bleue |
| 04 12 073 | 120h | 3 | 1 | 0 | 0 | 0 | 3 | 0.5 | 0 | 1 | Bleue |

| | | | | | | | | | | | |
|---|---|---|---|---|---|---|---|---|---|---|---|
| 0 indique soit l'absence de colonies isolées soit l'absence de fluorescence. Les intensités de fluorescence sont lues sur une échelle allant de 0 (aucune fluorescence) à 4 (fluorescence très intense). | | | | | | | | | | | |

### 4. INTERPRETATION

Un milieu selon l'invention, comprenant un substrat fluorogène, permettait la croissance de toutes les souches testées, et une détection aisée des légionelles.

### Exemple 5 :

Différentes souches du genre *Legionella* ont été testées sur un milieu selon l'invention comprenant un substrat chromogène d'alpha-glucosidase, l'alizarine-alpha-glucoside. La lecture des boîtes est ensuite effectuée après 72h et 96h d'incubation.

### 1. MILIEUX ET MICROORGANISMES

Le milieu de composition suivante a été utilisé (composition en g/l) :
- extrait de levure : 10 g/l
- acide alpha-cétoglutarique : 1 g/l
- ACES/KOH : 4/1,65 g/l
- Glycine : 3 g/l
- Cystéine hydrochloride : 0,4 g/l
- Pyrrophosphate ferrique : 0,25 g/l
- Glutathion : 5 g/l
- Agar: 17 g/l
- Octadecyl-silice : 1 g/l

Une solution-mère de substrat chromogène (alizarine-alpha-glucoside) était réalisée dans un solvant type DMSO à raison de 40g/l. Un volume correspondant à une concentration finale de 50mg/l de substrat a été ajouté dans ledit milieu en surfusion. De la même façon, une solution-mère de citrate de fer était réalisée dans de l'eau osmosée, puis la solution était filtrée avant d'être ajoutée dans le milieu à raison de 100mg/l.

### 2. ESSAIS

Les milieux ont été coulés en boîtes de Petri d'un diamètre de 55mm puis des souches de *Legionella* ont été ensemencées par isolement en trois cadrans à partir de suspensions à 0,5McFarland. Les boîtes ont été incubées à 37°C pendant 4jours.

### 3. RESULTATS

Les colonies formées ont été examinées visuellement après 72 et 96 heures d'incubation. Les colorations ainsi que les intensités ont été notées.

Les résultats sont consignés dans le tableau ci-après :

| | | ***Milieu T*** | | | | | | ***Milieu 1 50mg*/*l Alizarine-alpha-Glu + 100mg***/***l Citrate de fer*** | | | | | |
|---|---|---|---|---|---|---|---|---|---|---|---|---|---|
| | **Tps d**'**incub** | Croissance | | Intensité | | Couleur | | Croissance | | Intensité | | Couleur | |
| **Souches** | | M | C | M | C | M | C | M | C | M | C | M | C |
| *L. pneumophila* 07 10 144 | 72h | 3 | 0,5+ | 0 | 0 | 0 | 0 | 0 | 0 | 0 | 0 | 0 | 0 |
| | 96h | 3 | 0.75 | 0 | 0 | 0 | 0 | 0 | 0 | 0 | 0 | 0 | 0 |
| *L. pneumophila* 07 10 146 | 72h | 3 | 0.75 | 0 | 0 | 0 | 0 | 0 | 0 | 0 | 0 | 0 | 0 |
| | 96h | 3 | 1+ | 0 | 0 | 0 | 0 | 2 | 0 | 0.5 | 0 | R | 0 |
| *L. pneumophila* 07 10 142 | 72h | 3 | 1+ | 0 | 0 | 0 | 0 | 3 | 0.50 | 1 | 1 | R | R |
| | 96h | 3 | 1.25 | 0 | 0 | 0 | 0 | 3 | 0.75 | 1.5 | 1.5 | R | R |
| *L. pneumophila* 07 10 141 | 72h | 3 | 1 | 0 | 0 | 0 | 0 | 3 | 0.10 | 2 | 0 | RVi | 0 |
| | 96h | 3 | 1.25 | 0 | 0 | 0 | 0 | 3 | 0.75 | 2 | 1 | RVi | Rvi |
| *L. pneumophila* 07 10 139 | 72h | 3 | 0.75 | 0 | 0 | 0 | 0 | 2 | 0 | 1.5 | 0 | RVi | 0 |
| | 96h | 3 | 1 | 0 | 0 | 0 | 0 | 2 | 0 | 1.5 | 0 | Rvi | 0 |
| *L. jordanis* 04 09 055 | 72h | 3 | 1.25 | 0 | 0 | 0 | 0 | 3 | 0.10 | 2 | 0.5 | Vi | Vi |
| | 96h | 3 | 1.25 | 0 | 0 | 0 | 0 | 3 | 0.25 | 2 | 1 | Vi | Vi |
| *L. erythra* 04 12 069 | 72h | 3 | 0.75 | 0 | 0 | 0 | 0 | 0 | 0 | 0 | 0 | 0 | 0 |
| | 96h | 3 | 1+ | 0 | 0 | 0 | 0 | 0 | 0 | 0 | 0 | 0 | 0 |
| *L. rubrilucens* 04 12 075 | 72h | 3 | 1+ | 0 | 0 | 0 | 0 | 2 | 0 | 1 | 0 | R | 0 |
| | 96h | 3 | 1.25 | 0 | 0 | 0 | 0 | 2 | 0 | 1.5 | 0 | Vi | 0 |
| *L. longbeachae* 04 12 073 | 72h | 3 | 1+ | 0 | 0 | 0 | 0 | 0 | 0 | 0 | 0 | 0 | 0 |
| | 96h | 3 | 1.25 | 0 | 0 | 0 | 0 | 0.9 | 0.50 | 0.5 | 0 | R | 0 |
| *L. feelii* 04 12 070 | 72h | 3 | 1 | 0 | 0 | 0 | 0 | 3 | 0.10 | 0.5 | 0 | R | 0 |
| | 96h | 3 | 1+ | 0 | 0 | 0 | 0 | 3 | 0.25 | 0.5 | 0 | R | 0 |

| | | | | | | | | | | | | | |
|---|---|---|---|---|---|---|---|---|---|---|---|---|---|
| 0 indique soit l'absence de croissance, soit l'absence de colonies isolées soit l'absence de coloration. Les intensités de coloration sont lues sur une échelle allant de 0 (aucune coloration) à 4 (intensité très intense). Les couleurs varient de rose (noté R) à violet (Vi) en passant par Rvi (rose/violet). | | | | | | | | | | | | | |

### 4. INTERPRETATION

Un milieu selon l'invention, comprenant un substrat chromogène, permettait la croissance de 8/10 souches testées, et une détection aisée des légionelles via l'apparition d'une coloration rose à violette de la masse et des colonies.

## Revendications

1. Milieu réactionnel pour la culture, la détection et/ou l'identification de bactéries du genre *Legionella,* comprenant au moins un composé silicié, **caractérisé en ce que** ledit composé silicié est une silice apolaire.

2. Milieu réactionnel selon la revendication 1, **caractérisé en ce que** la concentration en silice apolaire est comprise entre 0,01 et 100 g/l, préférentiellement entre 1 et 5 g/l.

3. Milieu réactionnel selon l'une des revendications 1 ou 2, comprenant en outre un substrat enzymatique fluorogène ou chromogène.

4. Milieu réactionnel selon la revendication 3 dans lequel ledit substrat enzymatique fluorogène ou chromogène est un substrat d'oxydo-réductase ou d'hydrolase.

5. Milieu réactionnel selon l'une des revendications 1 à 4 comprenant en outre un mélange sélectif permettant la culture sélective de bactéries du genre *Legionella.*

6. Utilisation d'un milieu réactionnel comprenant au moins un composé silicié qui est une silice apolaire, pour la culture, la détection et/ou l'identification de bactéries du genre *Legionella.*

7. Utilisation selon la revendication 6 d'un milieu réactionnel dans lequel la concentration en composé silicié est comprise entre 0,01 et 100 g/l, préférentiellement entre 1 et 5 g/l.

8. Utilisation selon l'une des revendications 6 ou 7 d'un milieu réactionnel comprenant en outre un substrat enzymatique fluorogène ou chromogène.

9. Utilisation selon la revendication 8 d'un milieu réactionnel dans lequel ledit substrat enzymatique fluorogène ou chromogène est un substrat d'oxydo-réductase ou d'hydrolase.

10. Utilisation selon l'une des revendications 6 à 9 d'un milieu réactionnel comprenant en outre un mélange sélectif permettant la culture sélective des bactéries du genre *Legionella.*

11. Procédé de détection et/ou d'identification de bactéries du genre *Legionella,* comprenant les étapes suivantes :
a. mettre en contact un échantillon susceptible de contenir des bactéries du genre *Legionella* avec un milieu réactionnel selon l'une des revendications 1 à 5 ;
b. incuber, et
c. détecter la présence de bactéries du genre *Legionella.*

## Patentansprüche

1. Reaktionsmedium zum Kultivieren, Nachweisen und/oder Identifizieren von Bakterien der Gattung *Legionella,* das mindestens eine Siliziumverbindung umfasst, **dadurch gekennzeichnet, dass** es sich bei der Siliziumverbindung um ein unpolares Siliziumdioxid handelt.

2. Reaktionsmedium nach Anspruch 1, **dadurch gekennzeichnet, dass** die Konzentration an apolarem Siliziumdioxid zwischen 0,01 und 100 g/l, vorzugsweise zwischen 1 und 5 g/l, beträgt.

3. Reaktionsmedium nach einem der Ansprüche 1 oder 2, das weiterhin ein fluorogenes oder chromogenes Enzymsubstrat umfasst.

4. Reaktionsmedium nach Anspruch 3, wobei es sich bei dem fluorogenen oder chromogenen Enzymsubstrat um ein Substrat für die Oxidoreduktase oder Hydrolase handelt.

5. Reaktionsmedium nach einem der Ansprüche 1 bis 4, das weiterhin eine Selektionsmischung umfasst, die die selektive Kultur von Bakterien der Gattung *Legionella* gestattet.

6. Verwendung eines Reaktionsmediums, das mindestens eine Siliziumverbindung, bei der es sich um ein unpolares Siliziumdioxid handelt, umfasst, zum Kultivieren, Nachweisen und/oder Identifizieren von Bakterien der Gattung *Legionella.*

7. Verwendung nach Anspruch 6 eines Reaktionsmediums, bei dem die Konzentration an Siliziumverbindung zwischen 0,01 und 100 g/l, vorzugsweise zwischen 1 und 5 g/l, beträgt.

8. Verwendung nach einem der Ansprüche 6 oder 7 eines Reaktionsmediums, das weiterhin ein fluorogenes oder chromogenes Enzymsubstrat umfasst.

9. Verwendung nach Anspruch 8 eines Reaktionsmediums, bei dem es sich bei dem fluorogenen oder chromogenen Enzymsubstrat um ein Substrat für die Oxidoreduktase oder Hydrolase handelt.

10. Verwendung nach einem der Ansprüche 6 bis 9 eines Reaktionsmediums, das weiterhin eine Selektionsmischung, die die selektive Kultur von Bakterien der Gattung *Legionella* gestattet, umfasst.

11. Verfahren zum Nachweisen und/oder Identifizieren von Bakterien der Gattung *Legionella,* das die folgenden Schritte umfasst:
a. Inkontaktbringen einer Probe, die Bakterien der Gattung *Legionella* enthalten kann, mit einem Reaktionsmedium nach einem der Ansprüche 1 bis 5;
b. Inkubieren und
c. Nachweisen des Vorliegens von Bakterien der Gattung *Legionella.*

## Claims

1. A reaction medium for culturing, detecting and/or identifying bacteria of the *Legionella* genus, comprising at least one siliceous compound, **characterized in that** said siliceous compound is a nonpolar silica.

2. The reaction medium as claimed in claim 1, **characterized in that** the concentration of nonpolar silica is between 0.01 and 100 g/l, preferably between 1 and 5 g/l.

3. The reaction medium as claimed in either of claims 1 and 2, also comprising a fluorogenic or chromogenic enzymatic substrate.

4. The reaction medium as claimed in claim 3, in which said fluorogenic or chromogenic enzymatic substrate is an oxidoreductase substrate or a hydrolase substrate.

5. The reaction medium as claimed in one of claims 1 to 4, also comprising a selective mixture allowing the selective culture of bacteria of the *Legionella* genus.

6. Use of a reaction medium comprising at least one siliceous compound, which an apolar silica, for culturing, detecting and/or identifying bacteria of the *Legionella* genus.

7. The use as claimed in claim 6 of a reaction medium in which the concentration of siliceous compound is between 0.01 and 100 g/l, preferably between 1 and 5 g/l.

8. The use, as claimed in one of claims 6 or 7, of a reaction medium also comprising a fluorogenic or chromogenic enzymatic substrate.

9. The use, as claimed in claim 8, of a reaction medium in which said fluorogenic or chromogenic enzymatic substrate is an oxidoreductase substrate or a hydrolase substrate.

10. The use, as claimed in one of claims 6 to 9, of a reaction medium also comprising a selective mixture allowing the selective culture of bacteria of the *Legionella* genus.

11. A method for detecting and/or identifying bacteria of the *Legionella* genus, comprising the following steps:
a. bringing a sample that may contain bacteria of the *Legionella* genus into contact with a reaction medium as claimed in one of claims 1 to 5;
b. incubating, and
c. detecting the presence of bacteria of the *Legionella* genus.
